# EUROPÄISCHE PATENTANMELDUNG

(11) **EP 2 586 770 A1**
(43) Veröffentlichungstag der Anmeldung: **01.05.2013**
(21) Anmeldenummer: 11186815.4
(22) Anmeldetag: 27.10.2011
(51) Int. Cl.: C07C 263/10

(54) **Verfahren zur Herstellung von Isocyanaten und/oder Polyisocyanaten**

(71) Anmelder: BASF SE, 67056 Ludwigshafen (DE)
(72) Erfinder: Die Erfindernennung liegt noch nicht vor
(74) Vertreter: Rippel, Hans Christoph

(57) **Zusammenfassung**

Die Erfindung betrifft ein Verfahren zur Herstellung von Isocyanaten und/oder Polyisocyanaten durch Umsetzung der korrespondierenden Amine mit Phosgen, gegebenenfalls in Gegenwart eines Inertmediums, in einem Reaktor (1), wobei ein das Amin enthaltender erster Eduktstrom wird dem Reaktor (1) flüssig zugeführt wird und ein das Phosgen enthaltender zweiter Eduktstrom dem Reaktor gasförmig zugeführt wird. Der Reaktor ist ein Zentrifugalreaktor (1), der eine um eine zentrale Achse (7) rotierende Packung (9) in einem Gehäuse (13) aufweist, wobei der erste Eduktstrom und der zweite Eduktstrom der rotierenden Packung (9) so zugeführt werden, dass die Eduktströme aufgrund der Zentrifugalkraft in der rotierenden Packung (9) gemischt werden und nach außen transportiert werden, wobei durch das Mischen in der rotierenden Packung (9) das Phosgen mit dem Amin zum korrespondierenden Isocyanat oder Polyisocyanat reagiert.

## Beschreibung

Die Erfindung betrifft ein Verfahren zur Herstellung von Isocyanaten und/oder Polyisocyanaten durch Umsetzung der korrespondierenden Amine mit Phosgen, gegebenenfalls in Gegenwart eines Inertmediums, in einem Reaktor, wobei ein das Amin enthaltender erster Eduktstrom dem Reaktor flüssig zugeführt wird und ein das Phosgen enthaltender zweiter Eduktstrom dem Reaktor gasförmig zugeführt wird.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind. Dem gegenüber zeichnet sich eine Flüssigphasenphosgenierung dadurch aus, dass die Reaktion bei niedrigeren Temperaturen durchgeführt werden kann und eine Verdampfung der Edukte nicht erforderlich ist.

Bei herkömmlichen Flüssigphasenphosgenierungen wird ein aminhaltiger Eduktstrom in der Flüssigphase zugeführt. Dieser wird mit einem phosgenhaltigen Eduktstrom vermischt. Hierbei kann das Phosgen in einem inerten Lösungsmittel gelöst sein anschließend wird der phosgenhaltige Eduktstrom in eine Mischkammer eingespritzt, in der dieser mit dem aminhaltigen Eduktstrom vermischt wird. Das Amin und das Phosgen setzen sich unter Freisetzung von Chlorwasserstoff zu den entsprechenden Isocyanaten um.

Da das entstehende Isocyanat bei einer zu niedrigen Phosgenkonzentration mit dem überschüssigen Amin zu Harnstoff beziehungsweise anderen störenden, hochviskosen und festen Nebenprodukten reagiert, ist eine schnelle Vermischung des Amins mit dem Phosgen notwendig.

Um die Vorteile der Flüssigphasenphosgenierung mit denen der Gasphasenphosgenierung zu kombinieren, ist es aus WO-A 2008/006775 bekannt, die Amine in Form eines Aerosols mit dem Phosgen umzusetzen. Um eine hohe Eindringgeschwindigkeit des Phosgens in die flüssige aminhaltige Phase zu gewährleisten, sollten die Tröpfchen so klein wie möglich gehalten werden. Tropfengrößen des Amins liegen dabei im Bereich von 10 nm bis 1 mm. Hierbei ist jedoch weiterhin darauf zu achten, dass die Tropfengröße so gehalten wird, dass ein bei der Reaktion erzeugtes Aerosol von nachgeschalteten Tropfen-/Staubabscheidern abgeschieden werden kann. Die Erzeugung der Tropfen und damit des Aerosols kann zum Beispiel durch Düsen erfolgen. Nachteil des hier beschriebenen Verfahrens ist insbesondere die Herstellung des Aerosols und die anschließende Abtrennung der Tropfen aus dem Gasstrom.

Eine vollständige Gasphasenphosgenierung kann jedoch unerwünscht sein, insbesondere in Fällen, in denen das Amin bei Erhitzung zersetzt wird. Dies ist zum Beispiel dann der Fall, wenn als Ausgangssubstanzen Polyamine zur Herstellung von Polyisocyanaten eingesetzt werden. Diese lassen sich unter den Reaktionsbedingungen der Gasphasenphosgenierung nicht verdampfen.

Zur Durchführung einer Flüssigphasenphosgenierung, bei der ein flüssiger, aminhaltiger Strom und ein flüssiger, phosgenhaltiger Strom miteinander reagieren ist in CN 101104595 A ein Zentrifugalreaktor beschrieben. In diesem werden die flüssigen Eduktströme schnell gemischt und zur Reaktion gebracht. Nachteil der hier beschriebenen Flüssigphasenphosgenierung ist jedoch, dass die Ausbeute gering ist und ein höherer Energieverbrauch notwendig ist. Zudem ist ein höherer Hold-up an Phosgen erforderlich.

Aufgabe der vorliegenden Erfindung ist es, ein Verfahren zur Herstellung von Isocyanaten und/oder Polyisocyanaten bereit zu stellen, das die aus dem Stand der Technik bekannten Nachteile nicht aufweist.

Gelöst wird die Aufgabe durch ein Verfahren zur Herstellung von Isocyanaten und/oder Polyisocyanaten durch Umsetzung der korrespondierenden Amine mit Phosgen, gegebenenfalls in Gegenwart eines Inertmediums, in einem Reaktor, wobei ein das Amin enthaltender erster Eduktstrom dem Reaktor flüssig zugeführt wird und ein das Phosgen enthaltender zweiter Eduktstrom dem Reaktor gasförmig zugeführt wird, wobei der Reaktor ein Zentrifugalreaktor ist, der eine um eine zentrale Achse rotierende Packung in einem Gehäuse aufweist, wobei der erste Eduktstrom und der zweite Eduktstrom der rotierenden Packung so zugeführt werden, dass die Eduktströme aufgrund der Zentrifugalkraft in der Packung gemischt werden und nach außen transportiert werden, wobei durch das Mischen in der Packung das Phosgen mit dem Amin zum korrespondierenden Isocyanat oder Polyisocyanat reagiert.

Durch das erfindungsgemäße Verfahren werden zunächst die Vorteile der Gasphasenphosgenierung und der Flüssigphasenphosgenierung kombiniert. Durch die Zufuhr des Amins in flüssiger Phase kann die Reaktion bei niedrigeren Temperaturen durchgeführt werden als eine Gasphasenphosgenierung. Hierdurch ist es auch möglich, Amine zu phosgenieren, die der Gasphasenphosgenierung nicht zugänglich sind. Zudem kann durch das erfindungsgemäße Verfahren die Bildung von Nebenprodukten reduziert werden.

Der Einsatz des Zentrifugalreaktors hat zudem den Vorteil, dass keine Aerosoltröpfchen gebildet werden müssen, die nach durchgeführter Reaktion wieder aus dem Gasstrom entfernt werden müssen. Hierdurch werden auch die Nachteile der bekannten Gas/Flüssig-Phosgenierung, wie sie beispielsweise in WO-A 2008/006775 beschrieben ist, vermieden.

In einem Zentrifugalreaktor erfolgt die Trennung der Gasphase von der Flüssigphase am Austritt aus der rotierenden Packung aufgrund der Zentrifugalkraft.

Im Zentrifugalreaktor bilden sich sehr dünne Flüssigkeitsfilme auf der rotierenden Packung aus, die einen hohen Stoffaustausch zwischen der Gasphase und der Flüssigphase bewirken und daher positiv für die reaktive Umsetzung der Amine sind.

Weiterer Vorteil des erfindungsgemäßen Verfahrens ist es, dass mit dem Zentrifugalreaktor sehr kurze Verweilzeiten realisiert werden, was zu minimalen Ausbeuteverlusten und hohen Produktqualitäten führt. Unter einer hohen Produktqualität wird in diesem Zusammenhang ein Produkt mit guter Farbzahl, geringem Chlorgehalt hohen NCO-Zahlen, einer optimalen Molekulargewichtsverteilung etc. verstanden.

Die Reaktion wird vorzugsweise bei einem Druck im Bereich von 1 bis 20 bar (absolut), bevorzugt im Bereich 1 bis 10 bar (absolut) und besonders bevorzugt im Bereich von 1 bis 5 bar (absolut) durchgeführt. Die Temperatur liegt dabei vorzugsweise im Bereich von 50 bis 400°C, mehr bevorzugt im Bereich von 100 bis 300°C und besonders bevorzugt im Bereich von 150 bis 250°C.

Um in der rotierenden Packung des Zentrifugalreaktors eine ausreichend geringe Filmdicke zu erzielen, wird der Zentrifugalreaktor vorzugsweise so betrieben, dass im Kontaktbereich von Packung und Flüssigphase eine Zentrifugalbeschleunigung von 1 bis 5000 g, mehr bevorzugt eine Zentrifugalbeschleunigung im Bereich von 10 bis 1000 g und besonders bevorzugt eine Zentrifugalbeschleunigung im Bereich von 50 bis 250 g wirkt, wobei g die Erdbeschleunigung mit einem Wert von 9,81 m/s² ist.

Um die Bildung von Nebenprodukten bei der Reaktion zu vermeiden ist es vorteilhaft, das Phosgen im Überschuss zuzugeben. Durch die Zugabe des Phosgens im Überschuss wird vermieden, dass das Amin mit Chlorwasserstoff unter der Bildung eines Feststoffs reagiert. Der sich bei der Reaktion des Amins mit Chlorwasserstoff bildende Feststoff kann zwar phosgeniert werden, jedoch sind hierzu hohe Reaktionszeiten notwendig, was zu Ausbeuteverlusten bei der Phosgenierung führt, da die Verweilzeit der Feststoffe zu hoch ist.

Bei der Reaktion nicht umgesetztes Phosgen lässt sich ― gegebenenfalls nach einer Reinigung ― wieder zurückführen und zur Phosgenierung einsetzen. Bei der Reaktion entstandener Chlorwasserstoff kann ebenfalls gegebenenfalls nach einer Reinigung, zur Herstellung von Vinylchlorid oder zur Herstellung von Salzsäure eingesetzt werden. Alternativ ist es auch möglich, den Chlorwasserstoff nach dem sogenannten Deacon-Verfahren mit Sauerstoff zu Chlor umzusetzen und dieses in die Phosgensynthese zu rezyklieren.

Als Amine, die mit dem erfindungsgemäßen Verfahren zur Herstellung von Isocyanaten umgesetzt werden, können alle üblichen Amine eingesetzt werden. Dies sind beispielsweise aliphatische Diamine wie Hexamethylendiamin (HDA), Isophorondiamin (IPDA), sowie Toluylendiamin (TDA) und Methylendi(phenyldiamin) (MDA) im Gemisch mit seinen höheren Homologen. Insbesondere bei der Phosgenierung von MDA kann das erfindungsgemäße Verfahren besonders vorteilhaft eingesetzt werden. Hierbei wird besonders bevorzugt 4,4`-MDA eingesetzt. Dieses liegt im Allgemeinen in einer Mischung mit seinen isomeren Verbindungen 2,2`-MDA und 2,4`-MDA vor. Neben dem Einsatz von monomerem MDA kann auch Polymethylendi(phenyldiamin) (PMDA) mit dem erfindungsgemäßen Verfahren zum korrespondierenden Isocyanat umgesetzt werden. Da die Siedetemperatur von MDA bei mehr als 300°C liegt und sich PMDA nicht verdampfen lässt, ist das erfindungsgemäße Verfahren zur Phosgenierung dieser Amine zur Herstellung der korrespondierenden Isocyanaten besonders geeignet.

In einer Ausführungsform der Erfindung enthält der das Amin enthaltende erste Eduktstrom zusätzlich mindestens ein Lösungsmittel. Hierbei werden Lösungsmittel eingesetzt, die sich vor, während und nach der Umsetzung der Amine mit dem Phosgen inert verhalten. Geeignete Lösungsmittel sind zum Beispiel organische Lösungsmittel, beispielsweise aromatische Lösungsmittel, die auch halogeniert sein können. Geeignete Lösungsmittel sind zum Beispiel Toluol, Monochlorbenzol, o- oder p-Dichlorbenzol, Trichlorbenzol, Chlortoluol, Chlorxylol, Chlorethylbenzol, Chlornaphthalin, Chlordiphenyl, Xylol, Dekahydronaphthalin, Benzol und deren Mischungen. Weitere geeignete organische Lösungsmittel sind zum Beispiel Methylenchlorid, Perchlorethylen, Hexan, Diethylisophthalat, Tetrahydrofuran (THF), Dioxan, Trichlorfluormethan, Butylacetat und Dimethylformamid (DMF).

Durch den Einsatz der Lösungsmittel kann die Viskosität des flüssigen das Amin enthaltenden ersten Eduktstroms reduziert und hierdurch eine geringere Filmdicke erzeugt werden. Dies erlaubt eine schnellere Durchmischung des das Amin enthaltenden flüssigen Eduktstroms mit dem Phosgen. Zudem durchströmt die Flüssigkeit bei gleicher Drehzahl den Reaktor mit einer höheren Geschwindigkeit, wodurch die Verweilzeit weiter reduziert werden kann.

Das Phosgen wird bevorzugt im Überschuss bezogen auf die Amingruppen dosiert. Das molare Verhältnis von Phosgen zu Amingruppen beträgt vorzugsweise 1,01:1 bis 6:1, mehr bevorzugt 1,1:1 bis 4:1 und besonders bevorzugt 1,2:1 bis 3:1.

Ein erfindungsgemäß eingesetzter Zentrifugalreaktor zur Durchführung des Verfahrens weist üblicherweise eine erste Zuführung für den ersten Eduktstrom und eine zweite Zuführung für den zweiten Eduktstrom auf, wobei sowohl der erste Eduktstrom der Packung über die erste Zuführung und der zweite Eduktstrom der Packung über die zweite Zuführung zentral zugeführt werden. Durch die zentrale Zuführung wird erreicht, dass die gesamte radiale Ausdehnung der Packung zur Durchmischung von flüssigem Eduktstrom und gasförmigem Eduktstrom und damit zur Durchführung der Reaktion genutzt werden kann. Zudem wird durch die zentrale Zuführung erzielt, dass die Flüssigkeit in der Packung maximal beschleunigt wird.

Die Zuführung des ersten Eduktstroms und des zweiten Eduktstroms kann über zwei separate Zuführungen, die beispielsweise in Form von Rohrleitungen gestaltet sind, erfolgen. Alternativ ist es auch möglich, beispielsweise zwei konzentrische Rohre einzusetzen, wobei durch das innere Rohr der flüssige erste Eduktstrom und durch das äußere Rohr der gasförmige zweite Eduktstrom zugeführt werden. Alternativ ist es selbstverständlich auch möglich, dass durch das innere Rohr der gasförmige zweite Eduktstrom und das äußere Rohr der flüssige erste Eduktstrom zugeführt werden.

Der Vorteil einer konzentrischen Rohrleitung zur Zuführung der Eduktströme ist, dass hierdurch die Eduktströme gleichmäßig über den Umfang verteilt der Packung zugeführt werden und so eine gleichmäßige Belastung der rotierenden Packung erzielt wird.

Die Packung weist Kanäle auf, durch die Flüssigkeit und Gas strömen können. Aufgrund der Rotation der Packung bildet sich an den Wandungen der in der Packung ausgebildeten Kanäle ein Flüssigkeitsfilm aus.

Zur Ausbildung der Kanäle kann die Packung des Zentrifugalreaktors strukturiert oder unstrukturiert ausgeführt sein. In der Packung erfolgt die Vermischung der flüssigen mit der gasförmigen Phase. Die Gasphase ist vorzugsweise kontinuierlich. In der Packung bildet sich eine Filmströmung aus, wobei Flüssigkeit als Film auf den Oberflächen der Packung strömt und das Gas die freien Volumina der Kanäle durchströmt. Am Austritt der Packung, das heißt am Außenumfang der Packung ergibt sich eine Tropfenströmung, bei der die Flüssigkeit in Form von Tropfen aus der Packung austritt. Die Tropfen bewegen sich in der ebenfalls aus dem Reaktor austretenden Gasphase. Bevorzugt ist es, wenn die Kanäle in der Packung so gestaltet sind, dass die Packung porös ist und eine spezifische Oberfläche von mehr als 200 m²/m³ aufweist. Hierdurch wird eine große Fläche erzielt, auf der sich die Flüssigkeit als Film ausbilden kann und so ein hoher Durchsatz ermöglicht wird. Insbesondere gestattet eine poröse Packung eine gleichmäßigere Benetzung der Oberflächen und damit eine gleichmäßigere Reaktion.

Als Material für die Packung eignen sich zum Beispiel Metalle oder Keramiken. Diese sind gegenüber den bei der Reaktion auftretenden Temperaturen hinreichend beständig.

Die rotierende Packung des Zentrifugalreaktors kann zum Beispiel aus ungeordneten Fasern oder aus einer Kugelschüttung aufgebaut sein. Möglich sind jedoch auch Packungen in Form von Füllkörperschüttungen. Als Füllkörper können beliebige andere Formkörper eingesetzt werden. Als Formkörper eignen sich beispielsweise Zylinder, Ringe oder Berlsättel. Neben dem Einsatz von Formkörpern kann die rotierende Packung auch in Form von strukturierten Einbauten ausgebildet sein, beispielsweise als statischer Mischer oder Monolith oder in beliebiger anderer Form. Eine weitere Möglichkeit ist der Einsatz von offenporigen Schäumen.

Die rotierende Packung ist in einem feststehenden Gehäuse aufgenommen. Die die Packung verlassenden Tropfen werden aufgrund der Zentrifugalkraft nach außen geschleudert und prallen gegen das Gehäuse. Hier bildet sich ein Flüssigkeitsfilm an der Wandung des Gehäuses aus, der aufgrund der Schwerkraft nach unten strömt. Hier wird die Flüssigkeit gesammelt und kann über einen Flüssigkeitsaustrag aus dem Gehäuse entnommen werden.

Alternativ ist es auch möglich, die Packung zusätzlich mit einem Ring zu umschließen, wobei die die Packung verlassenden Tropfen in diesem Fall gegen den Ring prallen und vom Ring auf den Gehäuseboden abtropfen.

Auch in diesem Fall wird die Flüssigkeit auf dem Gehäuseboden gesammelt und über einen Flüssigkeitsauslass ausgetragen. Das Gas wird vorzugsweise an der Oberseite des Gehäuses aus dem Gehäuse entnommen. Hierdurch wird bereits im Reaktor eine Trennung in Gas und Flüssigkeit erzielt.

In einer bevorzugten Ausführungsform weist der Reaktor zusätzlich einen Inertgaseinlass aus. Durch den Inertgaseinlass kann dem Reaktor ein Inertgas zugeführt werden. Hierdurch lässt sich der Reaktor zum Beispiel mit einem Inertgas spülen, bevor der Reaktor in Betrieb genommen wird. Hierbei wird das Inertgas ebenfalls vorzugsweise über den Gasauslass, über den während des laufenden Betriebes das gasförmige nicht abreagierte Phosgen und der bei der Reaktion produzierte Chlorwasserstoff abgezogen wird, entnommen.

Der flüssige Produktstrom, der dem Reaktor entnommen wird, enthält üblicherweise das hergestellte Isocyanat, gegebenenfalls eingesetztes Lösungsmittel und geringe Mengen an Nebenprodukten. Die Nebenprodukte, die im Isocyanat verbleiben, können zum Beispiel durch eine zusätzliche Rektifikation oder Kristallisation in einem der Reaktion nachfolgenden Schritt von Isocyanat getrennt werden.

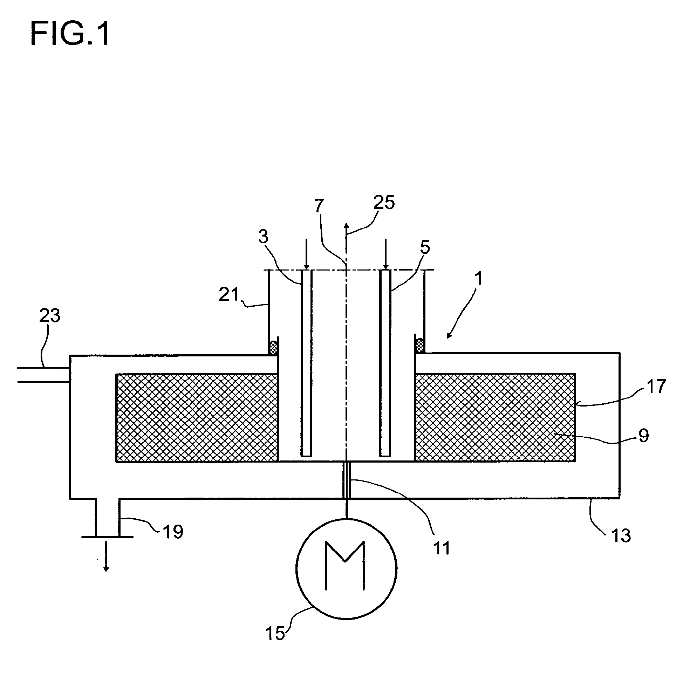

Ein Ausführungsbeispiel der Erfindung ist in der Figur dargestellt und wird in der nachfolgenden Beschreibung näher erläutert.

Die einzige Figur zeigt eine schematische Darstellung eines zur Herstellung von Isocyanaten eingesetzten Zentrifugalreaktors.

Einem Zentrifugalreaktor 1 wird über einen ersten Zulauf 3 ein erster Amin enthaltender Eduktstrom zugeführt. Über einen zweiten Zulauf 5 wird ein zweiter phosgenhaltiger, gasförmiger Eduktstrom zugeführt. Der erste Zulauf 3 und der zweite Zulauf 5 sind dabei so angeordnet, dass diese in einer um eine zentrale Achse 7 rotierenden Packung 9 münden. Aus dem ersten Zulauf 3 und dem zweiten Zulauf 5 gelangen die Eduktströme in die rotierende Packung 9. In der rotierenden Packung 9 sind Kanäle ausgebildet, an deren Wandungen sich aufgrund der Rotation der rotierenden Packung 9 und der damit verbundenen Zentrifugalkraft der flüssige erste Eduktstrom anlegt. An den Wandungen der rotierenden Packung 9 bildet sich auf diese Weise ein dünner Flüssigkeitsfilm aus, der in Kontakt mit dem Phosgen des zweiten Eduktstroms gelangt. Das Phosgen reagiert mit dem Amin in der Flüssigkeit unter Bildung von Isocyanat.

Die rotierende Packung 9 kann als geordnete oder ungeordnete Packung ausgebildet sein. Hierzu ist es zum Beispiel möglich, die rotierende Packung 9 in Form eines Käfigs zu gestalten, der mit Füllmaterial, beispielsweise Füllkörpern, gefüllt ist. Alternativ ist es auch möglich die Packung zum Beispiel aus Fasern oder Kugeln zu fertigen, wobei es bei einer Fertigung aus Kugeln bevorzugt ist, diese zu einer kompakten Packung zu sintern, wobei zwischen den einzelnen Kugeln die Kanäle ausgebildet werden. Besonders bevorzugt ist es, die rotierende Packung 9 als poröse Packung zu gestalten mit einer spezifischen Oberfläche von mehr als 200 m²/m³. Diese große spezifische Oberfläche führt zu einer entsprechend guten Flüssigkeitsverteilung innerhalb der rotierenden Packung 9 und damit einer geringen Filmdicke der Flüssigkeit auf den Wandungen der Packung. Durch die geringe Filmdicke wird der Stoffaustausch mit dem gasförmigen zweiten Eduktstrom weiter verbessert.

Um die rotierende Packung 9 anzutreiben ist diese über eine Welle 11 durch ein Gehäuse 13 mit einem Motor 15 verbunden. Um die gewünschten Drehzahlen zu erhalten ist es weiterhin möglich, dass dem Motor 15 ein Getriebe nachgeschaltet ist.

Die Drehzahl der rotierenden Packung 9 liegt vorzugsweise im Bereich von 50 bis 10.000 min⁻¹.

Aufgrund der großen Drehzahl der rotierenden Packung und der dadurch auftretenden Zentrifugalkräfte wird der flüssige Eduktstrom durch die rotierende Packung 9 zum äußeren Umfang beschleunigt. Am äußeren Umfang 17 der rotierenden Packung 9 bilden sich Flüssigkeitstropfen aus, die abreißen. Die Flüssigkeitstropfen prallen gegen die Wandung des Gehäuses 13 und laufen auf den Boden des Gehäuses 13 ab. Am Boden des Gehäuses 13 befindet sich ein Ablauf 19, durch den das flüssige Reaktionsgemisch abgezogen wird. Nicht abreagierte gasförmige Bestandteile, beispielsweise im Überschuss zugeführtes Phosgen sowie gasförmige Reaktionsprodukte wie Chlorwasserstoff werden vorzugsweise über einen zentralen Gasauslass 21 aus dem Zentrifugalreaktor 1 entnommen.

Um den Zentrifugalreaktor 1 insbesondere vor Inbetriebnahme und alternativ auch während des Betriebes mit einem Inertgas spülen zu können, weist das Gehäuse weiterhin einen Gaseinlass 23 auf.

In einer alternativen Ausführungsform ist es auch möglich, die rotierende Packung 9 mit einem Ring innerhalb des Gehäuses 13 zu umschließen, wobei der Ring als Prallring ausgeführt ist, gegen den die aus der rotierenden Packung 9 abreißenden Tropfen prallen. Am Prallring werden die Tropfen umgelenkt und zum Boden des Gehäuses 13 geleitet, so dass die Flüssigkeit über den Flüssigkeitsauslass 19 aus dem Zentrifugalreaktor 1 entnommen werden kann.

Die maximale Drehzahl der Packung wird vorzugsweise so gewählt, dass die abreißenden Tropfen eine Größe aufweisen, die eine einfache Abtrennung durch Aufprallen auf die Gehäusewand oder den Ring erlauben.

Die über den Auslass 19 entnommene Flüssigkeit kann in einem nachfolgenden Schritt aufgearbeitet werden. Insbesondere können flüssige oder feste Reaktionsnebenprodukte aus dem gewünschten Reaktionsprodukt, nämlich dem Isocyanat entfernt werden.

Das über den Gasauslass 21 entnommene Gas, was hier mit einem Pfeil 25 dargestellt ist, kann ebenfalls aufbereitet werden. Insbesondere ist es bevorzugt, das Abgas 25 in Phosgen, Chlorwasserstoff und gegebenenfalls Inertgas bzw. Lösungsmitteldampf aufzutrennen und das Phosgen und gegebenenfalls Lösungsmittel beispielsweise zurück in das Verfahren zu leiten. Der Chlorwasserstoff kann über den Umweg der Chlorsynthese zur Phosgenherstellung oder alternativ auch zur Salzsäureproduktion oder Vinylchloridproduktion eingesetzt werden.

Da das Amin dem Zentrifugalreaktor 1 in flüssiger Form zugeführt wird und nicht verdampft wird, eignet sich das erfindungsgemäße Verfahren insbesondere zur Durchführung der Aminphosgenierung zur Herstellung von Isocyanaten für Amine, die nicht verdampft werden können oder die eine sehr hohe Verdampfungstemperatur aufweisen. Besonders geeignet ist das erfindungsgemäße Verfahren zur Phosgenierung von MDA oder polymerem MDA zur Herstellung der jeweils korrespondierenden Isocyanate beziehungsweise Polyisocyanate.

### Bezugszeichenliste

- 1: Zentrifugalreaktor
- 3: erster Zulauf
- 5: zweiter Zulauf
- 7: zentrale Achse
- 9: rotierende Packung
- 11: Welle
- 13: Gehäuse
- 15: Motor
- 17: äußerer Umfang
- 19: Auslass
- 21: Gasauslass
- 23: Gaseinlass
- 25: Abgas

## Patentansprüche

1. Verfahren zur Herstellung von Isocyanaten und/oder Polyisocyanaten durch Umsetzung der korrespondierenden Amine mit Phosgen, gegebenenfalls in Gegenwart eines Inertmediums, in einem Reaktor (1), wobei ein das Amin enthaltender erster Eduktstrom dem Reaktor (1) flüssig zugeführt wird und ein das Phosgen enthaltender zweiter Eduktstrom dem Reaktor (1) gasförmig zugeführt wird, **dadurch gekennzeichnet, dass** der Reaktor ein Zentrifugalreaktor (1) ist, der eine um eine zentrale Achse (7) rotierende Packung (9) in einem Gehäuse (13) aufweist, wobei der erste Eduktstrom und der zweite Eduktstrom der rotierenden Packung (9) so zugeführt werden, dass die Eduktströme aufgrund der Zentrifugalkraft in der rotierenden Packung (9) gemischt werden und nach außen transportiert werden, wobei durch das Mischen in der rotierenden Packung (9) das Phosgen mit dem Amin zum korrespondierenden Isocyanat oder Polyisocyanat reagiert.

2. Verfahren gemäß Anspruch 1, **dadurch gekennzeichnet, dass** das Phosgen im Überschuss zugegeben wird.

3. Verfahren gemäß Anspruch 1 oder 2, **dadurch gekennzeichnet, dass** die Umsetzung bei einem Druck im Bereich von 1 bis 20 bar durchgeführt wird.

4. Verfahren gemäß einem der Ansprüche 1 bis 3, **dadurch gekennzeichnet, dass** die Umsetzung bei einer Temperatur im Bereich von 50 bis 400°C durchgeführt wird.

5. Verfahren gemäß einem der Ansprüche 1 bis 4, **dadurch gekennzeichnet, dass** der Zentrifugalreaktor (1) so betrieben wird, dass im Kontaktbereich von Packung und Flüssigphase eine Zentrifugalbeschleunigung von 1 bis 5000 g wirkt, wobei g die Erdbeschleunigung ist.

6. Verfahren gemäß einem der Ansprüche 1 bis 5, **dadurch gekennzeichnet, dass** der erste Eduktstrom der rotierenden Packung (9) über eine erste Zuführung (3) und der zweite Eduktstrom der rotierenden Packung (9) über eine zweite Zuführung (5) zentral zugeführt werden.

7. Verfahren gemäß einem der Ansprüche 1 bis 6, **dadurch gekennzeichnet, dass** die rotierende Packung (9) porös ist und eine spezifische Oberfläche von mehr als 200 m²/m³ aufweist.

8. Verfahren gemäß einem der Ansprüche 1 bis 7, **dadurch gekennzeichnet, dass** die rotierende Packung (9) aus einem Metall oder einer Keramik gefertigt ist.

9. Verfahren gemäß einem der Ansprüche 1 bis 8, **dadurch gekennzeichnet, dass** die rotierende Packung (9) aus ungeordneten Fasern, einer Füllkörperschüttung, aus einer Kugelschüttung, als Schaum oder in Form von strukturierten Einbauten aufgebaut ist.

10. Verfahren gemäß einem der Ansprüche 1 bis 9, **dadurch gekennzeichnet, dass** die rotierende Packung (9) von einem Ring umschlossen ist, durch den das durch die rotierende Packung (9) strömende Reaktionsgemisch zu einem Ablauf (21) umgelenkt wird.
